Europäisches Patentamt

European Patent Office

Office européen des brevets

(19)

(11) Publication number: **0 205 078 B1**

(12) **EUROPEAN PATENT SPECIFICATION**

(45) Date of publication of patent specification: **22.01.92**   (51) Int. Cl.5: **G01N 33/52**

(21) Application number: **86107526.5**

(22) Date of filing: **03.06.86**

(54) **Test strips and process for the production of test strips.**

(30) Priority: **11.06.85 DE 3520847**

(43) Date of publication of application:
**17.12.86 Bulletin  86/51**

(45) Publication of the grant of the patent:
**22.01.92 Bulletin  92/04**

(84) Designated Contracting States:
**DE FR GB IT NL SE**

(56) References cited:
**EP-A- 0 146 143       DE-A- 1 940 964**
**DE-A- 3 442 820       FR-A- 1 452 600**
**GB-A- 1 311 324       US-A- 4 774 054**

(73) Proprietor: **MILES INC.**
**1127 Myrtle Street**
**Elkhart Indiana 46514(US)**

(72) Inventor: **Engelmann, Helmut, Dipl.-Ing.**
**Tempelhofer Strasse 28**
**W-5090 Leverkusen 1(DE)**
Inventor: **Charlton, Steven, Dr.**
**1533 Strong Avenue, Elkhart**
**Indiana 46515(US)**

(74) Representative: **Dänner, Klaus, Dr. et al**
**c/o Bayer AG Konzernverwaltung RP Paten-**
**tabteilung**
**W-5090 Leverkusen 1 Bayerwerk(DE)**

EP 0 205 078 B1

## Description

The use of test strips in analysis and, in particular, in clinical chemistry belongs to the established processes. In comparison with conventional methods of wet chemistry, such analyses are faster and easier to carry out. Only test strips in which the reagent strip is applied and fixed to a flat carrier, that is to say the reagent strip projects out of the plane of the carrier, have previously been used.

However, when test strips are used in analysis, especially of whole blood, it is frequently necessary to wipe the strip. This wiping operation can lead to damage to the edges and surfaces of the reagent strip projecting out of the plane of the carrier. Subsequent evaluation, for example with a reflectance photometer, is thereby influenced and can lead to results which cannot be reproduced.

Exact positioning of the reagent strip on the carrier is also important for evaluation of the test strips with a reflectance photometer. If flat carriers are used for test strips, there is the risk that the reagent strip is brought out of the exact position as a result of inadequate adhesion of the adhesive.

Inadequate adhesion of the adhesive may exist, for example, if the adhesive used has not yet hardened completely during production and packaging, or the adhesive ages as a result of storage. Furthermore, an adhesive must always be used with flat carriers, which likewise can result in disadvantages.

Different adhesives must frequently be used in multiple strips in order to ensure compatibility of the reaction substances with the adhesive. Exact metering of the amount of adhesive presents difficulties and leads to indeterminate hardening of the test strips.

The influence of the residual solvent in the adhesive on the storage life of and change in the reaction substances introduced into the test strip is furthermore a great disadvantage.

Another disadvantage is that the adhesives can migrate into the reagent carrier in an uncontrolled manner. Absorption of a defined volume of sample by the reagent strip is thus no longer guaranteed, which leads to falsification of the analytical results.

The object of the invention was to develop test strips in which the reagent strips are largely protected from damage, in particular by wiping.

The invention relates to test strips consisting of a carrier (1) with one or more reagent strips (2), in which the carrier has the shape of a profile with depressions for accommodation of the reagent strips (2) and the reagent strips are fixed in these depressions. By sinking the reagent strips (2) in the carrier (1), the reagent strips (2) are well protected from damage such as may result, in particular, by the wiping operation. Exact positioning of the reagent strip (2) on the carrier (1) is likewise ensured.

The invention describes a test device comprising:

a) an elongated substrate having a first horizontal portion and a raised portion, said raised portion sloping upwardly from said first horizontal portion to a first termination point and then perpendicularly downward toward a second horizontal portion to form a right angle shoulder, said second horizontal portion further comprising a second termination point whereby said raised portion then slopes downwardly back to said first horizontal portion, whereby a wiping force moving axially across said first horizontal portion will first move gradually up to said second horizontal portion and then move gradually down substantially to the plane of said first horizontal portion, and

b) a reagent matrix positioned on a said second horizontal portion and abutting said raised portion at said right angle shoulder wherein the height of said reagent matrix is coextensive with said raised portion at said first and second termination points whereby the upper edge of said reagent matrix adjacent said raised portion is protected by the raised portion.

Further a process for the production of test strips is described - Drawings 1-10 show possible embodiments of the test strips according to the invention. As regards the drawings, specifically:

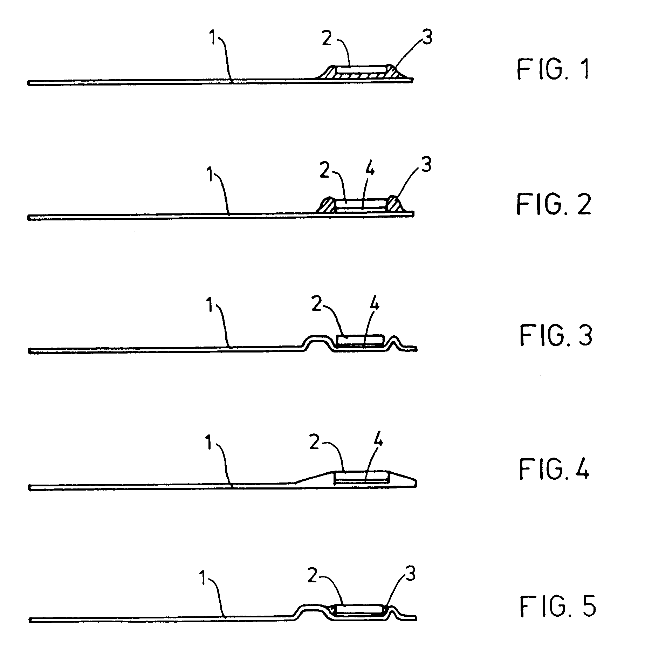

Figure 1: The reagent strip (2) is embedded in a hot melt adhesive or 2-component adhesive (3).

Figure 2: Reagent strips (2) are fixed to the carrier (1) with double-sided adhesive tape or adhesive (4) and then embedded with hot melt adhesive or 2-component adhesive (3).

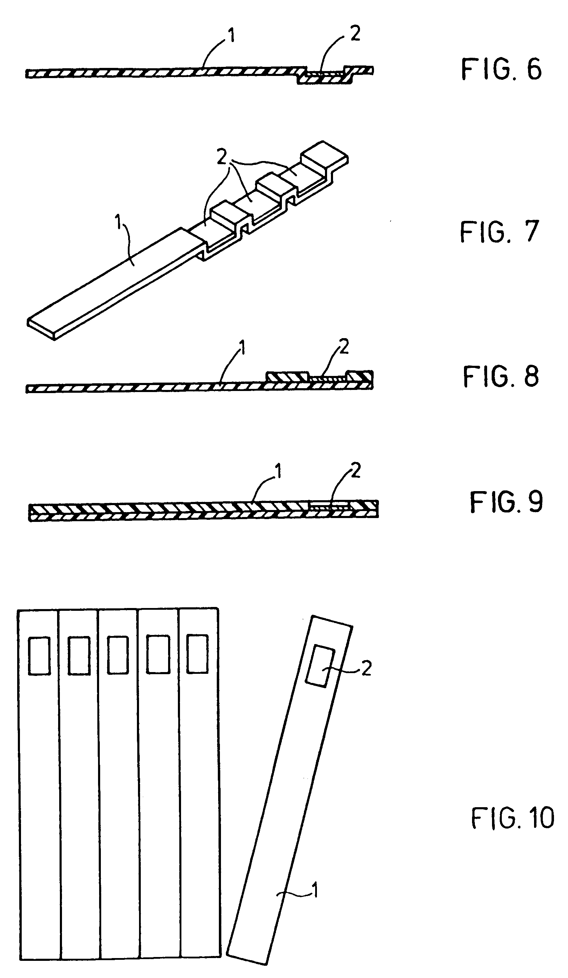

Figures 3-7: Various carrier profiles (1) produced by processes suitable for the particular carrier material, with the reagent strips (2) inserted. The reagent strips can be fixed by double-sided adhesive tape or adhesive (4). Embedding of the reagent strips with hot melt adhesive or 2-component adhesive (3) is likewise possible.

Figures 8 and 9: Carrier profiles (1) which have been produced by glueing together several layers, with depressions for accommodating the reagent strips (2).

Figure 10: Plan view of Figure 9.

The invention likewise relates to test strips in which the reagent strips (2) are fixed to a carrier (1) without an adhesive. The carrier (1) has the shape of a profile with one or more depressions for accommodating one or more reagent strips. The reagent strips are fixed to the carrier by shaping the carrier. This shaping can be effected mechanically, by pressure rolls, or by warming. A combination of both techniques is also possible. Shaping of the carrier preferably becomes by means of ultrasound. In this method, the carrier with the reagent strips laid in the depressions is guided past ultrasonic boosters. The edges of the depressions are shaped by the ultrasound such that they curve inwards and the reagent strip is held firmly by a type of "press fit". This technique operates without contact and adverse influence on the reagents in the reagent strip, such as, for example, the enzymes, is largely excluded.

Suitable carriers are materials which can be shaped, such as, for example, hard papers or plastics. Particularly suitable materials are thermoplastics, such as, for example, polyethylene, polypropylene, polyamides or polyesters. Any technique suitable for the particular carrier material can be used to produce the carrier profiles. In the case of carriers made of plastic, the profile is preferably produced by means of an extruder.

The reagent strips can consist of each material customary and known for the particular purpose, such as, for example, paper, cellulose and plastic.

The production process according to the invention is illustrated below in more detail with the aid of drawings 11-13.

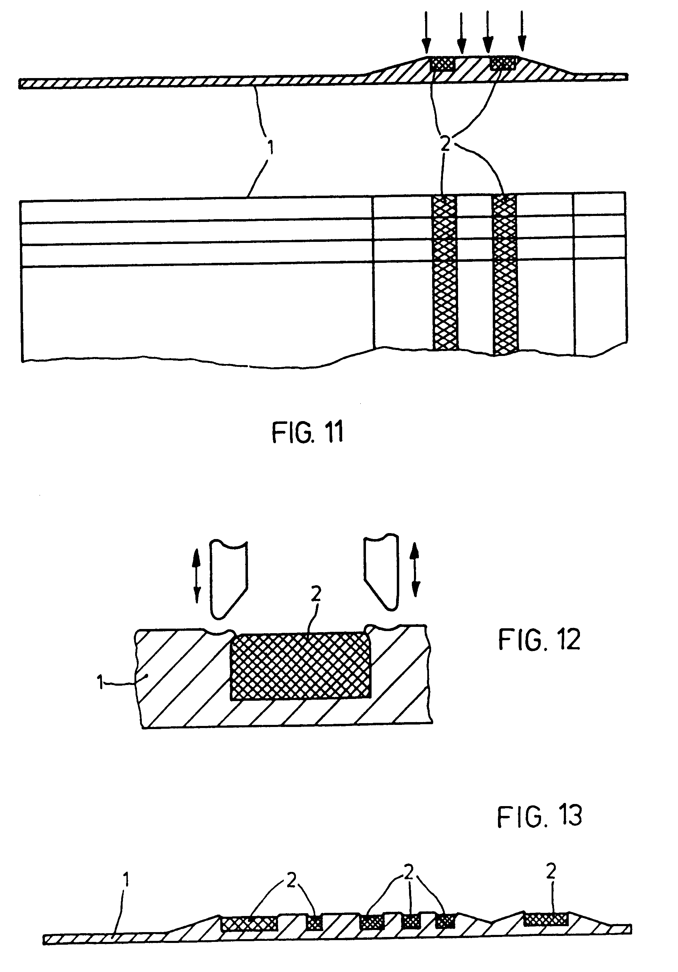

Figure 11 Carrier profile (1) with two reagent strips (2) pressed in without adhesive, in section, and the subsequent joint separation of reagent strips and carrier, in plan.

Figure 12 Shaping of the strip profiles (1) in the region of the reagent strip (2) pressed in, for example with ultrasound.

Figure 13 Cross-section of a carrier profile (3) for application of several reagent strips (4) - (9).

Figure 11 shows an extruded carrier profile (1) with two reagent strips (2) pressed in, without using adhesive. The carrier (1) is shaped in the region of the reagent strips (2) such that the reagent strip (2) lies protected and no damage to the surface can occur when the test strip is wiped and no fraying of the edges can occur during handling. This protected position of the reagent strip provides further advantages during packaging and during transportation. Contact and thus damage to the surface of the reagent carrier is prevented by the depression. In diagnostic applications, for example with blood, it is necessary to wipe off the blood corpuscles deposited on the surface. The possibility of damage to the surface during the wiping operation is considerably reduced by the depression. The surface is bridged by guiding the wiper over the upper limit of the carrier profile (1), and is therefore not touched and not damaged, and the edges are covered and cannot fray.

On the sides, the reagent strips pressed in are secured by shaping the carrier so that a firm connection is formed between the carrier (1) and reagent strip (2) without using an adhesive. The carrier (1) and the reagent strips are then cut together, by means of a cross-cutting knife, and made up.

## Claims

1. A test device comprising:

    a) an elongated substrate having a first horizontal portion and a raised portion, said raised portion sloping upwardly from said first horizontal portion to a first termination point and then perpendicularly downward toward a second horizontal portion to form a right angle shoulder, said second horizontal portion further comprising a second termination point whereby said raised portion then slopes downwardly back to said first horizontal portion, whereby a wiping force moving axially across said first horizontal portion will first move gradually up to said second horizontal portion and then move gradually down substantially to the plane of said first horizontal portion, and

    b) a reagent matrix positioned on a said second horizontal portion and abutting said raised portion at said right angle shoulder wherein the height of said reagent matrix is coextensive with said raised portion at said first and second termination points whereby the upper edge of said reagent matrix adjacent said raised portion is protected by the raised portion.

2. A test device according to claim 1 wherein the substrate is a plastic material.

3. A test device according to claim 1 in which the substrate is selected from polyethylene, polypropylene, polyamide and polyester.

## Revendications

1. Dispositif d'essai comprenant :
    a) un substrat allongé présentant une première partie horizontale et une partie surélevée, ladite partie surélevée s'élevant à l'oblique de ladite première partie horizontale jusqu'à un premier point terminal puis descendant perpendiculairement vers une seconde partie horizontale pour former un épaulement à angle droit, ladite seconde partie horizontale comprenant en outre un second point terminal où ladite partie surélevée redescend à l'oblique vers ladite première partie horizontale, une force d'essuyage traversant axialement ladite première partie horizontale montant d'abord progressivement jusqu'à ladite seconde partie horizontale, puis redescendant progressivement pratiquement jusqu'au plan de ladite première partie horizontale, et

b) une matrice de réactifs positionnée sur une dite seconde partie horizontale et étant en butée avec ladite partie surélevée audit épaulement à angle droit, la hauteur de ladite matrice de réactifs prolongeant la partie surélevée auxdits premier et second points terminaux, le bord supérieur de ladite matrice de réactifs adjacent à ladite partie surélevée étant protégé par la partie surélevée.

2. Dispositif d'essai selon la revendication 1, dans lequel le substrat est une matière plastique.

3. Dispositif d'essai selon la revendication 1, dans lequel le substrat est sélectionné parmi le polyéthyléne, le polypropylène, le polyamide et le polyester.

**Patentansprüche**

1. Testvorrichtung, umfassend:

(a) ein längliches Substrat mit einem ersten horizontalen Anteil und einem erhabenen Anteil, wobei der erhabene Anteil von dem horizontalen Anteil zu einem ersten Endpunkt ansteigt und anschliessend senkrecht zu einem zweiten horizontalen Anteil unter Ausbildung einer rechtwinkligen Kante abfällt, und der zweite horizontale Anteil zusätzlich einen zweiten Endpunkt aufweist, an dem der erhabene Anteil zum ersten horizontalen Anteil abfällt, wodurch ein Wischvorgang axial über den ersten horizontalen Anteil zunächst allmählich zum zweiten horizontalen Anteil hinaufgeführt und anschliessend allmählich im wesentlichen zur Ebene des ersten horizontalen Punktes hinabgeführt wird; und

(b) eine Reagensmatrix, die sich auf dem zweiten horizontalen Anteil befindet und an dem erhabenen Anteil an der rechtwinkligen Kante angrenzt, worin die Reagensmatrix die gleiche Höhe wie der erhabene Anteil am ersten und zweiten Endpunkt einnimmt, wodurch der obere Rand der Reagensmatrix, der an dem erhabenen Anteil angrenzt ist, durch den erhabenen Anteil geschützt ist.

2. Testvorrichtung nach Anspruch 1, worin das Substrat ein Plastikmaterial ist.

3. Testvorrichtung nach Anspruch 1, worin das Substrat ausgewählt ist aus Polyethylen, Polypropylen, Polyamid und Polyester.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13